Europäisches Patentamt

European Patent Office

Office européen des brevets

(11) Publication number: **0 009 930**
**B2**

# NEW EUROPEAN PATENT SPECIFICATION

(45) Date of publication of the new patent specification:
**24.08.88**

(51) Int. Cl.⁴: **C 12 N 15/00,** C 07 H 21/04 //
C12N9/02

(21) Application number: **79302019.9**

(22) Date of filing: **27.09.79**

(54) Recombinant DNA, method for preparing it and production of foreign proteins by unicellular hosts containing it.

(30) Priority: **10.10.78 US 950100**

(43) Date of publication of application:
**16.04.80 Bulletin 80/8**

(45) Publication of the grant of the patent:
**21.07.82 Bulletin 82/29**

(45) Mention of the opposition decision:
**24.08.88 Bulletin 88/34**

(84) Designated Contracting States:
**BE CH DE FR GB IT LU NL SE**

(56) References cited:
**Molecular Biology of the Genes, Watson, (3rd Ed. (Third Impression 1977)), Chapter 12, pp. 303 et seq. ("Involvement of RNA in Protein Synthesis")**
**Proc. Matl. Acad. SCi. USA, 75 (1978), No. 8, 3727-3731**
**Gene, 3,(1978), 65-71**
**Cell, 13, (1978), 65-71**
**Principles of Gene Manipulation, Old and Primrose, (2nd Ed.), 105, 112, 113**
**Nature, 275 (1978), 617-24**
**Cell, 15 (1978), 123-130**
**Proc. Natl. Acad. Sci. USA, 74 (1977), 3508-3512**
**Proc. Natl. Acad. Sci. USA, 73 (1976), 3900-3904**
**Proc. Natl. Acad. Sci. USA, 74(1977), 5255-5259**

(73) Proprietor: **THE BOARD OF TRUSTEES OF THE LELAND STANFORD JUNIOR UNIVERSITY, Encina 105 Stanford University, Stanford, California 94305 (US)**

(72) Inventor: **Nunber, Jack H., 4077 Amaranta, Palo Alto, California 94306 (US)**
Inventor: **Chang, Annie C.Y., 765 San Antonio Road, Palo Alto, California 94303 (US)**
Inventor: **Cohen, Stanley N., 271 Gabarda Way, Portola Valley, California 94025 (US)**
Inventor: **Schimke, Robert T., 639 Arastradero Road, Palo Alto, California 94306 (US)**

(74) Representative: **Allen, Oliver John Richard et al, Lloyd Wise, Tregear & Co. Norman House 105-109 Strand, London, WC2R 0AE (GB)**

## Description

### Field of the Invention

Recombinant DNA structures constructed by binding together segments of DNA derived from different biological sources have opened an area for production of a wide variety of only difficulty accessible proteins and providing for bacterial capability of a wide variety of functions, which are not native to the particular bacterium. For example, various eukaryotic enzymes and hormones may be produced by employing a eukaryotic gene specific for such hormone or enzyme, such as somatostatin and insulin. Alternatively, bacteria can be provided with the capability for the production of eukaryotic enzymes for performing chemical functions on a commercial scale.

One of the difficulties encountered in producing proteins foreign to the bacterial host is that in the utilization of signals recognized by the host species to accomplish synthesis of the protein, the protein product may be a covalently linked hybrid protein comprising the protein of the plasmic vector and the protein of the exogeneous gene. Unless there is some simple chemical or enzymatic technique for separating the two proteins, the product is not likely to be physiologically useful or economically efficient. Also, where the naturally occurring protein is a prodrug, means may be required for cleaving the inert portion from the active drug portion.

It is therefore desirable to find techniques which would allow for production of proteins, where the protein is produced in substantially native form and free of covalent bonding to other proteins.

### Description of the Prior Art

Shine and Dalgarno, Proc. Natl. Acad. Sci. U.S.A., 71, 1342 (1974) describe 3'-terminal sequence of E. coli 16S ribosomal RNA: complementarity to nonsense triplets and ribosome binding sites. Steitz and Steege, J. Mol. Biol. 114, 545 (1977) and Steege, Proc. Nat. Acad. Sci. U.S.A. 74, 4163 (1977) identify sequences on mRNA in the 5' direction from the initiator codon that are complementary to the CCUCC sequence at the 3' end of the 16S ribosomal RNA species proposed to be involved in the binding of mRNA to ribosomes.

Komaroff, et al., Proc. Natl. Acad. Sci., U.S.A. 75, 3727–3731 (1978) reports the preparation of a fused protein consisting of insulin and penicillinase. Rat insulin cDNA was inserted into the PstI site of plasmid pBR322 and the recombinant plasmid used to transform E. coli. The PstI site lies in the penicillinase structural gene of the plasmid.

The objective of the workers of this paper was to insert a eukaryotic structural gene into a plasmid in such a way as to obtain expression of the gene in a transformed bacterium, and in particular insertion of the structural information for insulin into the penicillinase gene of the plasmid to provide for expression of the insulin sequence as a fusion product which would be transported outside the cell. The experimental results confirmed the presence of a penicillinase-insulin hybrid poly peptide, i.e. a fused protein.

Backmann and Ptashne, Cell 13 65–71 January 1978 and Mann et al, Gene 3 (1978) 97–112, both demonstrate the expression of a prokaryotic gene in a prokaryotic host. There is no evidence in these two references to support any wide implication or teaching extending to the expression of eukaryotic genes.

According to the present invention there is provided a method for producing in a prokaryotic unicellular host a protein foreign to the host, characterised in that a functional recombinant DNA structure consisting essentially of:

(1) a first DNA sequence from a eukaryotic source expressing the protein,

(2) a second DNA sequence comprising a vector from a source compatible with the host and which does not exchange information with the eukaryotic source, the vector having an intact replicon and promoter for the first DNA sequence, and

(3) a third DNA sequence between the first and second DNA sequences encoding for a simulated ribosomal binding site recognised by the host adjacent a deoxyribonucleotide triplet encoding for methionine on the first DNA sequence, wherein the third DNA sequence and the triplet define a start site such that initiation of translation occurs at the triplet, is used to transform the host whereby the host achieves the capability of expressing the first DNA sequence, and producing the foreign protein free from vector proteins.

According to a second aspect of the invention, there is provided a functional recombinant DNA structure for use in the method of the invention, the structure consisting essentially of:

(1) a first DNA sequence from an eukaryotic source expressing a protein,

(2) a second DNA sequence comprising a vector from a second source, compatible with a prokaryotic unicellular host and which does not exchange information with the eukaryotic source, the vector having an intact replicon and promoter for the first DNA sequence, and

(3) a third DNA sequence between the first and second DNA sequences encoding for a ribosomal binding site recognised by the host adjacent a deoxyribonucleotide triplet encoding for methionine on the first DNA sequence, wherein the third DNA sequence and the triplet define a start site such that initiation of translation occurs at the triplet in a prokaryotic unicellular host transformed by the functional recombinant DNA structure.

According to a third aspect of the invention there is provided a method for preparing the functional recombinant DNA structure of the invention, the method comprising annealing and/or ligating

(1) a first DNA sequence from a eukaryotic source expressing the protein,

(2) a second DNA sequence comprising a vector from a second source which does not exchange information with the eukaryotic source, the vector having an intact replicon and promoter for the first DNA sequence and being compatible with a prokaryotic unicellular host, wherein a third DNA sequence is introduced between the first and second DNA sequences prior to or concomitantly with the annealing and/or ligating, the third DNA sequence encoding for a ribosomal binding site recognised by the host, and being introduced adjacent a deoxyribonucleotide triplet encoding for methionine on the first DNA sequence, the third DNA sequence and the triplet defining a start site such that initiation of translation occurs at the triplet in a prokaryotic unicellular host transformed by the functional recombinant DNA structure.

Summary of the Invention

Method and compositions are provided for the production of a foreign eukaryotic protein by a prokaryotic, unicellular host such as a bacterial host. The method employs a vector comprising a replicon having an intact replicator locus and capable of replication in said host, preferably in conjunction with a gene which allows for survival selection, an intact promoter and a DNA sequence that ordinarily is not able to be propagated in said host. The foreign DNA sequence has proximate to the 3'-terminus of the coding strand a codon which when transcribed defines the initiating methionine codon of the sequence and is preferably modified with a nucleotide oligopolymer which when transcribed simulates a prokaryotic ribosomal binding site to provide initiation of protein synthesis. The termini of the DNA sequence of the vector modified with an oligopolymer complementary to said oligopolymer defines the prokaryotic ribosomal binding site. The modified vector and modified foreign DNA coding sequence are annealed to form a replicating structure which is employed in the transformation or transfection of said bacterial host, whereby the transformed host is capable of expressing said foreign DNA sequence to produce foreign protein.

Description of the specific embodiments

Methods and compositions are provided involving inserting DNA foreign to a host capable of complete protein expression into a vector having free ends where foreign DNA is provided at the 3'-terminus of the coding strand with an oligopolymer which when transcribed defines the prokaryotic 16S ribosomal binding site in proximity to a nucleotide triplet which when transcribed defines the initiator methionine codon to define a protein synthesis starting site. The termini, usually 3', of the vector are extended with an oligopolymer of nucleotides complementary to the above oligopolymer to provide cohesive termini.

The modified foreign gene and modified vector are combined and annealed so as to form a structure capable of replication (propagation) in the host strain. The resulting plasmid need not be ligated, ligation apparently occurring in vivo, but may be introduced directly into the bacterium under transforming conditions. The bacterium may then be cloned, so that the foreign DNA sequence may be expressed, and the structure replicated. Once the structure has been replicated by the bacterium, it need no longer be prepared in vitro.

The term vector intends a DNA sequence having an intact or competent replicon and promoter, so that it is capable of replication and transcription to provide mRNA. The vector may be derived from a plasmid, virus or phage which is compatible with the bacterial host.

The foreign DNA structure is derived from a source which does not exchange genetic information with the host. The foreign DNA structure is derived from a eukaryotic source. The coding strand intends the strand for transcription or coding for mRNA. The other strand will be referred to as the anticoding or nonsense strand.

Since the plasmid can be used as exemplary of the structure of the recombinant DNA sequence employed for transformation, except that the plasmid and virus are circular, while other DNA sequences derived from phage need not be circular, for the most part the recombinant DNA sequence will be referred to as a plasmid. It should be understood that in referring to a recombinant plasmid, the plasmid is only exemplary and is not limiting. Similarly, as to the foreign DNA sequence, the gene will be used as exemplary of any DNA which provides a useful product and derived from a eukaryotic source which does not exchange information with the bacterial host.

In referring to the DNA sequences, defining shall intend when transcribed to mRNA, the function of the resulting sequence of ribonucleotides.

The foreign DNA sequence will desirably have a phenotypical trait and may be modified by the introduction at the 3' termini of an oligopolymer of nucleotides defining a simulated prokaryotic ribosomal binding site, with the terminus being at or adjacent to a nucleotide triplet defining an initiating methionine codon to define a start site. The DNA employed as a vector is also modified at the termini, usually 3', by introducing an oligopolymer having complementary nucleotides to those introduced on the coding strand, so as to serve as cohesive ends for annealing to the modified foreign gene. The modified foreign gene and the modified vector are combined and annealed providing a DNA structure capable of replication in the host species. The ends may be ligated to form covalent bonds or the DNA structure e.g. plasmid, may be used directly without ligation in transformation (includes transfection) of a host bacterium. Alternatively, the oligopolymer sequence may be blunt end ligated to the vector and foreign DNA sequence. The bacteria may then be cloned to provide expression of the foreign gene with the protein product being free of proteins from the replicon.

## Foreign DNA

The foreign DNA may have one or more DNA sequences, e.g. genes, depending upon the manner in which it was prepared or obtained and the purpose of the transformation. The double stranded DNA is derived from eukaryotic cells, the source being one which does not normally exchange genetic information with the bacterial host. The fragments employed will generally have molecular weights in the range of 0.01 to $20 \times 10^6$d, usually in the range of 0.1 to $10 \times 10^6$d.

By introducing one or more exogenous DNA segments into a unicellular organism, the organism will be able to produce polypeptides and proteins ("poly(amino acids)") which the organism could not previously produce. In some instances the poly(amino acids) will have utility in themselves, while in other situations, particularly with enzymes, the enzymatic product(s) will either be useful in itself or useful to produce a desirable product.

One group of poly(amino acids) which are directly useful are hormones. Illustrative hormones include parathyroid hormones, growth hormone, gonadotropins (FSH, luteinizing hormone, chorionogonadatropin, and glycoproteins), insulin, ACTH, somatostatin, prolactin, placental lacto gen, melanocyte stimulating hormone, thyrotropin, parathyroid hormone, calcitonin, enkephalin, and angiotensin.

Other poly(amino acids) of interest include serum proteins, fibrinogin, prothrombin, thromboplastin, globulin e.g. gamma-globulins or anti bodies, heparin, antihemophilia protein, oxytocin, albumins, actin, myosin, hemoglobin, ferritin, cytochrome, myoglobin, lactoglobulin, histones, avidin, thyroglobulin, interferon, kinins transcortion, and peptide antigens for use in making vaccines.

Where the genes or genes encode for one or more enzymes, the enzymes may be used for fulfilling a wide variety of functions. Included in these functions are nitrogen fixation, production of amino acids e.g. polyiodothyronine, particularly thyroxine, vitamins, both water and fat soluble vitamins, antimicrobial drugs, chemotherapeutic agents e.g. antitumor drugs, polypeptides and proteins e.g. enzymes from apoenzymes and hormones from prohormones, diagnostic reagents, energy producing combinations e.g. photosynthesis and hydrogen production, prostaglandins, steroids, cardiac glycosides and coenzymes.

The enzymes may be individually useful as agents separate from the cell for commercial applications, e.g. in detergents, synthetic transformations and diagnostic agents. Enzymes are classified by the I.U.B. under the classification as I. Oxidoreductases; II. Transferases; III. Hydrolases; IV. Lyases; V. Isomerases; and VI. Ligases.

The foreign DNA sequence may be obtained in a wide variety of ways. Conveniently, messenger RNA (mRNA) may be isolated and by employing reverse transcriptase, the DNA can be synthesized. This method has the advantage of providing for a relatively short fragment of DNA, a relatively large source of genetic material, and frequently the presence of the triplet defining the methionine initiator codon. Alternatively, one can synthesize either the mRNA or the DNA, either synthesizing the complete sequence de novo or synthesizing fragments and then allowing the fragments to be covalently joined with the appropriate polymerase or ligase enzyme. Alternatively, with the appropriate genetic material, one could employ endonuclease cleavage, where the restriction enzyme cleaves at a site adjacent to the nucleotide sequence defining the methionine initiator codon.

In the event that the nucleotide sequence defining the initiator methionine codon is cleaved from the gene, the appropriate nucleotide bases may be added to provide such sequence, followed by the addition of the bases or oligopoly mer to provide the sequence defining the ribosomal binding site or the nucleotides added as a unit. It should be appreciated that while the normal nucleotide triplet on the strand that defines the methionine codon is 5'-dCAT base triplets other than AUG on the mRNA may in particular situations serve to be translated as methionine e.g. GUG, so that other nucleotide triplets on the sense strand may find use. Desirably, the restriction enzyme employed for isolating the gene from other genetic material will be chosen to provide for the desired nucleotide triplet adjacent the sense strand 3'-terminus.

## Vector

In the preparation of the vector or replicon, double stranded plasmid, viral or phage DNA is cleaved with an appropriate restriction enzyme to provide for an intact replicator locus and system, and promoter. If a plasmid, the plasmid chosen will be capable of replicating in a microorganism, particularly a bacterium, which is susceptible to transformation. Various unicellular microorganisms can be transformed, such as bacteria, fungii, plants and algae. That is, those unicellular organisms which are capable of being grown in cultures or by fermentation. Bacteria, which are susceptible to transformation, include members of the Enterobacteriaceae, such as strains of Escherichia coli; Salmonella; Bacillacea, such as Bacillus subtilis; Pneumococcus; Streptococcus, and Haemophilus influenzae.

After cleavage of the plasmid, depending upon the nature of the restriction enzyme, either square ends or slanted ends (cleavage at different but adjacent sites) may be obtained. Restriction enzymes which provide square ends include Hae III and Alu I, and enzymes providing slant ends include PstI and Hpa II. As indicated previously, a plasmid need not be used, but rather the DNA may be derived from a virus or phage which is compatible with the bacterial host which is to be transformed the viral DNA will be treated in the same manner as plasmid DNA for providing the vector.

Desirably, the vector is obtained by endonuclease (restriction enzyme) cleavage of the vector source, where the site for cleavage at the 5′-terminus has as the next successive base complementary base to the 3′-terminal base of the oligopolymer defining the simulated prokaryotic ribosomal binding site. In effect, one wishes to recreate the endonuclease cleavage site. In this manner the recircularized plasmid may be cleaved to regenerate the modified gene at its 3′-terminus employing the same endonuclease. Also, a preferable cleavage site is between the A and G of a CAG sequence.

The two different portions of the DNA used to prepare the recombinant plasmid will then be modified to provide the nucleotides defining the ribosomal binding site or complementary oligopolymer. Where the triplet defining the methionine initiator codon has been removed from the foreign gene, the appropriate triplet, as well as other bases defining appropriate amino acids may be added to the DNA using the appropriate enzyme e.g. ligase. The sequence defining the simulated prokaryotic ribosomal binding site may then be added to the DNA adjacent to, but not necessarily joined to, the triplet defining the initiator methionine codon. The ribosomal binding site has been described in Shine and Dalgarno, supra. Since the binding site on the ribosome has a plurality of cytosines, the nucleotides defining the start site on the coding strand of the DNA will also have a plurality of cytosines, so that the messenger RNA, which is complementary to the DNA will have a plurality of guanosines. The cytosine enriched tail defining the ribosomal binding site may be achieved by forming an oligohomopolymer of cytosines employing a transferase, or alternatively, a fragment which is similar or identical to that defining the 16S ribosomal RNA binding site may be synthetically prepared and then bonded to two of the termini of the DNA employing a ligase.

While conveniently, the deoxyribonucleotides defining the simulated ribosomal binding site may be ligated as a unit or added individually to the 3′-termini of the foreign DNA sequence, the same final result of providing for a DNA sequence defining a start site may be achieved in many different ways. For example, a ds DNA segment defining the ribosomal binding site, having the triplet defining the methionine codon as appropriate may be blunt or staggered end ligated between the insert (foreign DNA) and the vector.

The need for introduction of the ribosomal binding site to the DNA will be present whenever the genetic material is prepared synthetically, or in the preparation or isolation of the gene, the ribosomal binding site has been altered or removed.

In order to provide cohesive ends, the vector will be modified by providing tails complementary to the tails added to the foreign gene. That is, for example, where a poly-d-cytosine has been conjugated to the 3′ ends of the foreign gene, a poly-dguanosine will be added to the 3′ ends of the vector.

The number of members on the coding strand defining the ribosomal binding site will vary depending on whether an identical complementary unit is provided or the binding site is only partially complementary, as in the case of a homopoly mer. Usually, there will be on the average at least three base members, and not more than 50 base members, more usually from 8 to 40, and generally from 10 to 25 base members. Furthermore, on the average, the complementary tail attached to the vector need not be of the same length as the length of the tail added to the coding strand, usually being at least one-half the number of bases, and may be equal to or slightly greater than the number of bases defining the simulated ribosomal binding site, particularly when the number of bases in the tail attached to the foreign gene is in the low portion of the range.

Conveniently, the bases on the coding strand defining the simulated ribosomal binding site is immediately adjacent the codon complementary to the initiator methionine complementary codon. However, interruption is permissible. The interruption should not exceed twenty bases and preferably not exceed nine bases. The bases defining the simulated ribosomal binding site plus the bases intervening between the simulated ribosomal binding site and the bases defining the initiator methionine codon, which together define a start site, may be in phase or out of phase with the vector preferably out of phase.

Desirably, the vector should have a phenotypical property which allows for selection. Particularly, are those genes which provide for survival selection, for example, by providing for resistance to antibiotics or heavy metals or polypeptides, which have bacteriostatic or bacteriocidal functions. Alternatively, a host can be chosen which lacks an appropriate growth factor, which can be supplied by a gene on the replicon.

After preparation of the two double stranded DNA sequences, the foreign gene and vector are combined for annealing and/or ligation to provide for a functional recombinant DNA structure. With plasmids, the annealing involves the hydrogen bonding together of the cohesive ends of the vector and the foreign gene to form a circular plasmid which has cleavage sites. The cleavage sites are then normally ligated to form the completely closed and circularized plasmid.

The annealing, and as appropriate, recircularization can be performed in whole or in part in vitro or in vivo. Preferably, the annealing is performed in vitro. The annealing requires an appropriate buffered medium containing the DNA fragments. The temperature employed initially for annealing will be 40 to 70°C, followed by a period at lower temperature, generally from 10 to 30°C. The molar ratio of the two segments will generally be in the range of 1–5:5–1. The particular temperature for annealing will depend upon the binding strength of the cohesive termi. While 0.5 hr to 2 or more days may be employed for annealing, it is believed that a period of 0.5 to 6 hrs may be sufficient. The time employed for the annealing will vary with the temperature employed, the nature of the salt solution, as well as the nature of the cohesive termini.

The ligation, when in vitro, can be achieved in conventional ways employing DNA ligase. Ligation is conveniently carried out in an aqueous solution (pH6–8) at temperatures in the range of 5 to 40°C. The concentration of the DNA will generally be from 10 to 100 g/ml. A sufficient amount of the DNA ligase or other ligating agent e.g. $T_4$ ligase, is employed to provide a convenient rate of reaction, generally ranging from 5 to 50U/ml. A small amount of a protein e.g. albumin, may be added at concentrations of 10 to 200 g/ml. The ligation with DNA ligase is carried out in the presence of magnesium at 1–10 mM.

At the completion of the annealing or ligation, the solution may be chilled and is ready for use in transformation.

Various techniques exist for transformation or transfection of a bacterial cell with plasmid DNA.

The transformed bacteria may then be cloned in accordance with conventional methods, particularly employing the phenotypical property for selection. The resulting clones are then selected for their capability to produce the functional protein and used for expression of the desired protein in a form free of protein derived from the vector and replication of the plasmid.

Experimental

(All temperatures not otherwise indicated are in centigrade. The following abbreviations are employed with their indicated meanings: 2ME-2-mercaptoethanol; SSC – saline sodium citrate; DHFR – dihydrofolate reducatase; cDNA – complementary DNA; TBE – tris-borate EDTA; PABA – Penassay broth agar.)

Example 1

Preparation of pBR332 plasmid with DHFR cDNA insert.

DNA complementary to DHFR mRNA was synthesized essentially as described by Buell et al, Biol. Chem. 253, 2471 (1978), using avian myeloblastosis virus (AMV) reverse transcriptase and polysomal RNA obtained by indirect immunoprecipitation of DHFR-synthesizing polysomes from methotrexate-resistant AT-3000 S-180 mouse cells [Alt et al, Biol. Chem., 253, 1357 (1978)]. The RNA had been estimated to contain DHFR mRNA as 20% of its mRNA. The reaction was carried out in 100 μl [50 mM Tris, pH 8.2 at 42°, 140 mM KCl, 10 mM MgCl₂, 30 mM 2ME, 100 μl/ml oligodT (12–18) (Collaborative Research)], 500 μM each deoxynucleotide triphosphate (dNTP) [dCTP was adjusted to ~4Ci/mm with 32P-dCTP)], 340 μg polysomal RNA (estimated to contain 5 μg polyA-RNA), and 45 units AMV reverse transcriptase. The reaction was incubated at 42° for 30 min and stopped by the addition of 0.25 M EDTA pH8.0 to 10 mM.

Approximately 1.4 μg cDNA was synthesized.

To the reaction mixture was added E. coli tRNA (30 μg), followed by extraction with an equal volume of phenol (saturated with TEN) (2X), followed by ether, before being passed over a Sephadex G-50 fine column (0.7×20 cm) in 10 mM Tris pH 7.4, 2 mM EDTA, 10 mM NaCl (TEN). The void volume was collected and precipitated with 2.5 volumes of ethanol. After centrifugation, the cDNA was dissolved in 200 μl TEN containing 110 mm NaOH and heated at 70° for 25 min to hydrolyze the RNA, followed by cooling, neutralization with equimolar Hcl (buffered with Tris, pH 7.4 to 40 mM) made to 0.4 M NaCl and precipitated with ethanol.

After centrifugation, the cDNA was dissolved in 50 μl 5 mM Tris pH 7.4, 0.1 mM EDTA and then used as template for the synthesis of the second strand by E. coli DNA polymerase I essentially as described in Seeburg et al, Nature, 270, 486 (1977). (The reaction was carried out at 42° for 10 minutes in 100 μl (50 mM Tris pH 8.2, 20 mM KCl, 7 mM MgCL₂, 10 mM 2 ME, 0.1 mM EDTA) using 1.1 μg cDNA, 10 units E. coli DNA polymerase I, and 200 μM of each dNTP with dCTP adjusted to 30 ci/mM as above. Approximately 0.85 μg of the second strand was synthesized. The reaction was stopped and extracted as above before being passed over a Sephadex G-50 fine column (0.5×7 cm) in TEN containing only 0.1 mM EDTA. Column fractions containing the ds cDNA were adjusted to 40 mM NaOAc pH 4.5, 300 mM NaCl, 3 mM Zn(OAc)₂ and then treated with Aspergillus oryzae S1 nuclease [5 U/ml as described by Wickens et al, J. Biol. Chem. 253, 2483 (1978)]. Digestion was carried out at 37° for 60 min and stopped by the addition of EDTA to 10 mM.

After extraction with phenol and ether (as previously described) and adjusted to 0.3 M NaCl, followed by precipitation with ethanol, approximately 1.0 μg ds cDNA was obtained. Aliquots of a) first strand product, b) first strand product after base treatment, c) second strand product and d) second strand product after S1 nuclease treatment were examined on a 1.5% agarose gel under alkaline conditions as described by McDonnel et al, J. Mol. Biol., 110, 119 (1977).

Terminal addition of dCTP to the ds cDNA by terminal deoxynucleotidyl transferase [TdT, Chang and Bollum, J. Biol. Chem. 246, 909 (1971)] was carried out by a modification of the Co⁺⁺ procedure [Roychoudhury, et al, Nuc. Acids Res., 3, 101 (1976)]. The reaction was performed in 500 μl containing 140 mM cacodylic acid, 30 mM Tris base, 100 mM KOH (final pH 7.6), 0.1 mM dithiothreitol, 150 μm dCTP [adjusted to 8 Ci/mm with ³H-dCTP (Amersham)], 1 mM CoCl₂ (added to prewarmed reaction mix prior to enzyme addition), approximately 1.0 μg ds cDNA (assuming a number average MW of approximately 600 base pairs, this provides 10 pM 3′ (termini/ml) and 0.5 μl TdT (2.3×10⁵ units/ml). The reaction was allowed to proceed at 37° for 10 minutes before being cooled and sampled to determine incorporation. Approximately 30 dC residues were added per 3′ terminus. The reaction was stopped (EDTA to 10 mM), extracted, desalted and precipitated with ethanol as above.

Aliquots of e) second strand product, f) second strand product after S1 nuclease treatment and g) dC-tailed ds cDNA were analyzed on a 1.7% agarose gel in Tris-acetate-NaCl. The dC-tailed ds cDNA was then preparatively electrophoresed on a similar gel and the "1500 base pair" region cut out of the gel and electrophoretically eluted into a dialysis bag as described (see McDonnel et al, supra). The eluted material was extracted as above, concentrated by lyophilization and recipitated with ethanol. After centrifugation, the '1500 base pair' dC-tailed ds cDNA (approximately 80 ng) was redissolved in 10 mM Tris HCl pH 7.4, 0.25 mM EDTA, 100 mM NaCl (annealing buffer).

pBR322 plasmid DNA, isolated as described (Kuperstock and Helinski, Biochem. Biophys. Res. Commun., 54, 1451 (1973) was digested with a 1.5 fold excess of PstI endonuclease under conditions suggested by the vendor (New England Biolabs) and the linear plasmid DNA was cut out and eluted as described above from a 0.7% agarose gel in TBE [Sharp and Sambrook, Biochemistry, 12, 3055 (1974)]. The plasmid DNA was 'tailed' with dG residues following the procedures described above. Approximately 15–20 dG residues were added per 3' terminus. Following extraction with phenol and ethanol as previously described, the dG-tailed vector was passed over a Sephadex G-50 file column (0.5×7 cm) in annealing buffer and the void volume was collected. Equimolar amounts of dC-tailed ds cDNA and dG-tailed vector DNA were allowed to anneal essentially as described in Sanger and Coulson, FEBS Lett., 87, 107 (1978), except that the vector concentration was kept at 75 ng/ml in the annealing reaction. Circularization was monitored by electron microscopy and was typically about 20–40%. This annealed DNA was used directly for transformation into χ1776 or χ2282.

Example 2

Transformation and cloning with DHFR containing plasmid

pBR322 plasmid DNA that had been annealed in vitro with dc tailed DHFR cDNA (designated 1°) was introduced into χ1776 or χ2282, using a modification of a previously described transfection procedure [Enea et al, J. Mol. Biol., 96, 495 (1975)]. One ml of an overnight bacterial culture was inoculated into 100 ml of L broth supplemented with diaminopimelic acid (DAP, 50 µg/ml) and (for χ1776 only) thymidine (4 µg/ml). Bacterial cultures were grown until exponential phase at 35° and then harvested by centrifugation at 4°. Cells were washed in 0.3 volume 10 mM NaCl, resuspended in 30 ml freshly prepared MCN buffer (70 mM MnCl$_2$; 40 mM sodium acetate, pH 5.6 and 30 mM calcium chloride) and chilled on ice for 20 minutes. Cells were collected, resuspended in 1 ml MCN and added in 200 µl aliquots to 50 µl DNA in TEN (10 mM Tris-HCl, pH 7.5; 0.1 mM EDTA, 50 mM NaCl) or MCN buffer. After chilling at 0° for 30 minutes, reactions were incubated at 27° for 5 min, chilled again for 30 min, and 50 µl samples were plated onto Penassay broth agar supplemented with DAP, thymidine (for χ1776), and antibiotics as indicated. When χ2282 was used, the selective medium was M9 minimal agar supplemented with 0.5% casamino acids, biotin (2 µg/ml), DAP (50 µg/ml) and trimethoprim (Tp) (2.5–10 µg/ml) plus tetracycline (Tc) or kanamycin (Km) as indicated in the table. Plates containing transformants were incubated at 32° and colonies were scored 2 to 3 days after plating. pBR322 plasmid DNA lacking the cDNA insert was used as a control. cDNA preparations labeled as 2° consisted of plasmid DNA isolated from a non-fractionated population of clones that had previously been transformed with chimeric molecules carrying a cDNA insert.

Table 1

| | Host | Transforming DNA | | Transforming/ngDNA | |
|---|---|---|---|---|---|
| | | Plasmid Vector | Insert | Tc 5µg/ml | Tc 10µg/ml |
| A. | χ1776 | pBR322 | none | $4\times10^2$ | $2\times10^2$ |
| B. | χ1776 | pBR322 | cDNA(1°) | $6\times10^1$ | $3.2\times10^1$ |
| C. | χ2282 | pBR322 | cDNA(1°) | $7\times10^1$ | – |
| D. | χ2282 | pBR322 | cDNA(2°) | $6\times10^1$ | – |
| E. | χ | pDHFR7 | | $7.5\times10^1$ | – |

| | Tc/Tp 5µg/ml of each | In situ hybridization with DHFR cDNA probe (% positive) |
|---|---|---|
| A. | $<2\times10^{-3}$ | – |
| B. | – | 40 |
| C. | $2\times10^0$ | 44 |
| D. | $1.3\times10^{-1}$ | – |
| E. | $2.5\times10^1$ | – |

## Example 3

Detection of colonies containing DHFR cDNA inserts by in situ hybridization

Colonies were screened for DHFR sequences using a modification of an in situ hybridization procedure [Grundstein and Hagness, Natn. Acad. Sci. U.S.A. 72, 3961 (1975)]. Tc-resistant colonies were transferred to nitro-cellulose filters (Millipore, HAWG) that had been placed on PABA plates containing Tc (10 µg/ml). (Filters had been washed twice by boiling in H₂O and autoclaved prior to placing on plates). After 2–3 days of bacterial growth at 32°, the filter was removed from the plate and placed on a Whatman #3 pad saturated with 0.5N NaOH. After 7′, the filter was sequentially transferred to a series of similar pads saturated with 1M Tris pH 7.4 (twice, 7′ each); 1.5 M NaCl, 0.5 M Tris, pH 7.5 (once, 7′); and 0.30 M NaCl, 0.03 M Nacitrate (2×SSC), (once, 7′). After the excess liquid was removed by suction the filter was placed on a pad containing 90% ethanol, dried by suction and backed in vacuo at 80° for 2 hrs.

Prior to hybridization, filters were pretreated for 3–6 hrs at 65° in hybridization buffer that contained 5×SSC pH 6.1, 0.2% sodium dodecyl sulfate (SDS), 0.02% Ficol 400 (Pharmacia) and 8 µg/ml E. coli tRNA. Hybridizations were performed with individual filters in 1.5 ml hybridization buffer containing 2×10⁴ cpm ³²P-labelled purified DHFR cDNA [Alt et al, Biol. Chem., 253, 1357 (1978)] in a sealed plastic bag at 65° for 24 hours. The filters were then washed in hybridization buffer (once, 60′ at 65°); in 5×SSC, pH 6.1 (three times, 60′ each at 65°); and in 2×SS, pH 7.4 (twice, 10′ at room temperature); air dried, and prepared for autoradiography. A collection of χ1776 colonies were shown to contain a DHFR cDNA insert.

## Example 4

Inhibitor analysis of DHFR from bacterial cells

Stationary phase cultures of χ2282 expressing trimethoprim resistance were grown in the presence of Tp (1 µg/ml) in minimal medium, washed with isotonic saline, and suspended in 50 mM potassium phosphate buffer pH 7.0 containing 10 mM benzamidine and 10 mM phenyl methyl sulfonyl fluoride (3 volumes buffer to 1 volume cells). The suspension was sonicated and centrifuged at 10,000 rpm for 15 min. The supernatant was centrifuged for 1 hr at 100,000 xg before being studied. An R₂ methotrexate resistant mouse cell extract was prepared. Enzyme activity was measured by the radioactive folic acid assay previously described [Alt et al, J. Biol. Chem. 251, 3063 (1976)]. Protein was determined by the method of Lowry. Approximately 3 units of activity from the χ2282 extract or 5 units from the methotrexate resistant mouse cell extract were incubated with inhibitor for 10 min at 24° before assaying for folate reductase activity; background values were determined by measuring enzyme activity in the presence of 10 mM methotrexate. The results for enzyme activity were closely comparable in the presence of varying concentrations of trimethoprim for the mouse abstracts and χ2282 as evidenced by the amount of enzyme needed to reduce 1 nM of folate in 15 min at 37°.

The subject method provides for the preparation of a functional protein, such as an enzyme, free of protein or polypeptide chain from the plasmid vector which might interfere with or significantly change the character of the enzyme from the eukaryotic source. Thus, the subject invention provides a method for introducing a DNA sequence defining a ribosomal binding site adjacent a triplet defining an initiator methionine codon to provide a unit which allows for ribosomal initiation of protein production at the initial site of a DNA sequence foreign to the host. In this manner, the difficult problem of separating the covalently bound vector protein from the foreign protein is avoided.

Although the foregoing invention has been described in some detail by way of illustration and example for purposes of clarity of understanding, it will be obvious that certain changes and modifications may be practiced within the scope of the appended claims.

## Claims

1. A method for producing in a prokaryotic unicellular host a protein foreign to the host, characterised in that a functional recombinant DNA structure consisting essentially of:

(1) a first DNA sequence from a eukaryotic source expressing the protein,

(2) a second DNA sequence comprising a vector from a source compatible with the host and which does not exchange information with the eukaryotic source, the vector having an intact replicon and promoter for the first DNA sequence, and

(3) a third DNA sequence between the first and second DNA sequences encoding for a simulated ribosomal binding site recognised by the host adjacent a deoxyribonucleotide triplet encoding for methionine on the first DNA sequence, wherein the third DNA sequence and the triplet define a start site such that initiation of translation occurs at the triplet,

is used to transform the host whereby the host achieves the capability of expressing the first DNA sequence, and producing the foreign protein free from vector proteins.

2. A method according to Claim 1, characterised in that the third DNA sequence is an oligohomopolymer of d-cytosine of from 3 to 40 nucleotides and is out of phase with the vector.

3. A method according to Claim 1 or Claim 2, characterised in that the first DNA sequence encodes an enzyme.

4. A method according to Claim 1 or Claim 2, characterised in that the vector is obtained by endonuclease cleavage of a plasmid to provide staggered ends.

5. A functional recombinant DNA structure suitable for use in the method of Claim 1, characterised in that the structure consists essentially of:

(1) a first DNA sequence from an eukaryotic source expressing a protein,

(2) a second DNA sequence comprising a vector from a second source, compatible with a prokaryotic unicellular host and which does not exchange information with the eukaryotic source, the vector having an intact replicon and promoter for the first DNA sequence, and

(3) a third DNA sequence between the first and second DNA sequences encoding for a ribosomal binding site recognised by the host adjacent a deoxyribonucleotide triplet encoding for methionine on the first DNA sequence, wherein the third DNA sequence and the triplet define a start site such that initiation of translation occurs at the triplet in a prokaryotic unicellular host transformed by the functional recombinant DNA structure.

6. A recombinant DNA structure according to Claim 5, characterised in the third DNA sequence is an oligohomopolymer of d-cytosine out of phase with the vector.

7. A recombinant DNA structure according to Claim 6, characterised in that the third DNA sequence has from 8 to 25 d-cytosines.

8. A recombinant DNA structure according to any one of Claims 5 to 7, characterised in that the vector is formed by endonuclease cleavage of a plasmid to provide staggered ends.

9. A method for preparing the functional recombinant DNA structure defined in any one of Claims 5 to 8 characterised in that the method comprises annealing and/or ligating

(1) a first DNA sequence from a eukaryotic source expressing the protein,

(2) a second DNA sequence comprising a vector from a second source which does not exchange information with the eukaryotic source, the vector having an intact replicon and promoter for the first DNA sequence and being compatible with a prokaryotic unicellular host,

wherein a third DNA sequence is introduced between the first and second DNA sequences prior to or concomitantly with the annealing and/or ligating, the third DNA sequence encoding for a ribosomal binding site recognised by the host, and being introduced adjacent a deoxyribonucleotide triplet encoding for methionine on the first DNA sequence, the third DNA sequence and the triplet defining a start site such that initiation of translation occurs at the triplet in a prokaryotic unicellular host transformed by the functional recombinant DNA structure.

**Patentansprüche**

1. Verfahren zum Herstellen eines für den Wirt fremden Proteins in einem prokaryontischen einzelligen Wirt, dadurch gekennzeichnet, dass eine funktionelle rekombinante DNA-Struktur bestehend im wesentlichen aus:

(1) einer ersten DNA-Sequenz aus einer das Protein ausdrückenden eukaryontischen Quelle,

(2) einer zweiten DNA-Sequenz, die einen Vektor aus einer Quelle aufweist, die mit dem Wirt kompatible ist und mit der eukaryontischen Quelle keine Information austauscht, wobei der Vektor ein intaktes Replikon und einen intakten Promoter für die erste DNA-Sequenz besitzt, und

(3) einer dritten, zwischen der ersten und zweiten DNA-Sequenz liegenden DNA-Sequenz, die auf eine simulierte ribosomale vom Wirt zu erkennende Bindungsstelle neben einem Desoxyribonukleotiden-Triplett kodiert, das bei der ersten DNA-Sequenz auf Methionin kodiert, wobei die erste DNA-Sequenz und das Triplett einen Startpunkt definieren, so dass die Initiierung der Translation am Triplett einsetzt, dazu verwendet wird, den Wirt umzuwandeln, wodurch der Wirt die Fähigkeit erwirbt, die erste DNA-Sequenz auszudrücken, und das fremde Protein produziert wird, das von Vektorproteinen frei ist.

2. Verfahren nach Anspruch 1, dadurch gekennzeichnet, dass als dritte DNA-Sequenz ein Oligohomopolymer des d-Cytosin aus 3 bis 40 Nukleotiden verwendet wird, das mit dem Vektor nicht in Phase liegt.

3. Verfahren nach einem der vorhergehenden Ansprüche 1 oder 2, dadurch gekennzeichnet, dass die erste DNA-Sequenz ein Enzym kodiert.

4. Verfahren nach einem der vorhergehenden Ansprüche 1 oder 2, dadurch gekennzeichnet, dass der Vektor durch Endonuklease-Spaltung eines Plasmids erzielt wird, um gestaffelte Enden vorzusehen.

5. Funktionelle rekombinante DNA-Struktur geeignet zur Verwendung in dem Verfahren nach Anspruch 1, dadurch gekennzeichnet, dass die Struktur im wesentlichen aus

(1) einer ersten DNA-Sequenz aus einer ein Protein ausdrückenden eukaryontischen Quelle,

(2) einer zweiten DNA-Sequenz, die einen Vektor aus einer Quelle aufweist, die mit dem Wirt kompatible ist und mit der eukaryontischen Quelle keine Information austauscht, wobei der Vektor ein intaktes Replikon und einen intakten Promoter für die erste DNA-Sequenz besitzt, und

(3) einer dritten, zwischen der ersten und zweiten DNA-Sequenz liegenden DNA-Sequenz besteht, die auf eine simulierte ribosomale vom Wirt zu erkennende Bindungsstelle neben einem Desoxyribonukleotiden-Triplett kodiert, das bei der ersten DNA-Sequenz auf Methionin kodiert, wobei die erste DNA-Sequenz und das Triplett einen Startpunkt definieren, so dass die Initiierung der Translation am Triplett in einem prokaryontischen einzelligen Wirt einsetzt, der durch die funktionelle rekombinante DNA-Struktur umgewandelt wurde.

6. DNA-Struktur nach Anspruch 5, dadurch gekennzeichnet, dass als dritte DNA-Sequenz ein Oligohomopolymer des d-Cytosin verwendet wird, das mit dem Vektor nicht in Phase liegt.

7. DNA-Struktur nach Anspruch 6, dadurch gekennzeichnet, dass die dritte DNA-Sequenz von 8 bis 25 d-Cytosins aufweist.

8. DNA-Struktur nach einem der vorhergehenden Ansprüche 5 bis 7, dadurch gekennzeichnet, dass der Vektor gebildet wird durch Endonuklease-Spaltung eines Plasmids, um gestaffelte Enden vorzusehen.

9. Verfahren zum Darstellen der funktionellen rekombinanten DNA-Struktur nach den Ansprüchen 5 bis 8, gekennzeichnet durch Spannungsfreimachen (annealing) und/oder Ligatieren (ligating)

(1) einer ersten DNA-Sequenz aus einer das Protein ausdrückenden eukaryontischen Quelle und

(2) einer zweiten DNA-Sequenz, die einen Vektor aus einer zweiten Quelle aufweist, die mit der eukaryontischen Quelle keine Information austauscht, wobei der Vektor ein intaktes Replikon und einen intakten Promoter für die erste DNA-Sequenz besitzt und mit dem prokaryontischen einzelligen Wirt kompatible ist, und dadurch, dass vor oder begleitend mit dem Spannungsfreimachen und/oder Ligatieren zwischen der ersten und der zweiten DNA-Sequenz eine dritte DNA-Sequenz eingeführt wird, die auf eine vom Wirt zu erkennende ribosomale Bindungsstelle kodiert und neben einem bei der ersten DNA-Sequenz auf Methionin kodierenden Desoxyribonukleotid-Triplett eingeführt wird, wobei die dritte DNA-Sequenz und das Triplett einen Startpunkt definieren, so dass die Initiierung der Translation am Triplett in einem einzelligen, durch die funktionelle rekombinante DNA-Struktur umgewandelten prokaryontischen Wirt einsetzt.

## Revendications

1. Procédé de production chez un hôte unicellulaire procaryotique d'une protéine étrangère à cette hôte, caractérisé en ce qu'on utilise une structure d'ADN de recombinaison fonctionnel comprenant essentiellement:

(1) une première séquence d'ADN provenant d'une source eucaryotique exprimant la protéine,

(2) une seconde séquence d'ADN comprenant un vecteur provenant d'une source compatible avec l'hôte et qui n'échange pas d'information avec la source eucaryotique, ce vecteur comportant un réplicon intact et un promoteur intact pour la première séquence d'ADN, et

(3) une troisième séquence d'ADN entre la première séquence d'ADN et la seconde séquence d'ADN, codant pour un site de liaison ribosomique simulé reconnu par l'hôte au voisinage d'un triplet de désoxyribonucléotides codant pour la méthionine situé sur la première séquence d'ADN, la troisième séquence d'ADN et le triplet définissant un site d'initiation tel que l'initiation de la traduction a lieu au niveau du triplet, pour transformer l'hôte, de façon que l'hôte devienne capable d'exprimer la première séquence d'ADN et de produire la protéine étrangère dépourvue de protéine de vecteur.

2. Procédé selon la revendication 1, caractérisé en ce que la troisième séquence d'ADN est un oligo-homopolymère de d-cytosine de 3 à 40 nucléotides et est déphasée par rapport au vecteur.

3. Procédé selon la revendication 1 ou 2, caractérisé en ce que la première séquence d'ADN code pour une enzyme.

4. Procédé selon la revendication 1 ou 2, caractérisé en ce qu'on obtient le vecteur par coupure par endonucléase d'un plasmide pour obtenir des extrémités décalées.

5. Structure d'ADN de recombinaison fonctionnel convenant pour être utilisée dans le procédé selon la revendication 1, caractérisée en ce que ladite structure comprend essentiellement:

(1) une première séquence d'ADN provenant d'une source eucaryotique exprimant une protéine,

(2) une seconde séquence d'ADN comprenant un vecteur provenant d'une seconde source, compatible avec un hôte unicellulaire procaryotique et qui n'échange pas d'information avec la source eucaryotique, le vecteur comportant un réplicon intact et un promoteur intact pour la première séquence d'ADN, et

(3) une troisième séquence d'ADN entre la première séquence d'ADN et la seconde séquence d'ADN, codant pour un site de liaison ribosomique reconnu par l'hôte adjacent à un triplet de désoxyribonucléotides codant pour la méthionine situé sur la première séquence d'ADN, la troisième séquence d'ADN et le triplet définissant un site d'initiation de telle façon que l'initiation de la traduction a lieu au niveau du triplet chez un hôte unicellulaire procaryotique transformé par la structure d'ADN de recombinaison fonctionnel.

6. Structure d'ADN de recombinaison selon la revendication 5, caractérisée en ce que la troisième séquence d'ADN est un oligo-homopolymère de d-cytosine déphasé par rapport au vecteur.

7. Structure d'ADN de recombinaison selon la revendication 6, caractérisée en ce que la troisième séquence d'ADN comporte 8 à 25 d-cytosines.

8. Structure d'ADN de recombinaison selon l'une quelconque des revendications 5 à 7, caractérisée en ce que le vecteur est formé par coupure par endonucléase d'un plasmide pour obtenir des extrémités décalées.

9. Procédé de préparation de la structure d'ADN de recombinaison fonctionnel selon l'une quelconque des revendications 5 à 8, caractérisé en ce que ledit procédé consiste à condenser et/ou lier

(1) une première séquence d'ADN provenant d'une source eucaryotique exprimant la protéine,

(2) une seconde séquence d'ADN comprenant un vecteur provenant d'une seconde source, qui n'échange pas d'information avec la source eucaryotique, le vecteur comportant un réplicon intact et un promoteur intact pour la première séquence d'ADN et étant compatible avec un hôte unicellulaire procaryotique, en introduisant une troisième séquence d'ADN entre la première séquence d'ADN et la seconde, avant la fusion et/ou la liaison ou en même temps, cette troisième séquence d'ADN codant pour un site de liaison ribosomique reconnu par l'hôte et étant introduite au voisinage d'un triplet de désoxyribonucléotides codant pour la méthionine situé sur la première séquence d'ADN, la troisième séquence d'ADN et le triplet définissant un site d'initiation de telle façon que la traduction a lieu au niveau du triplet chez un hôte unicellulaire procaryotique transformé par la structure d'ADN de recombinaison fonctionnel.